**Europäisches Patentamt**

**European Patent Office**

**Office européen des brevets**

(19)

(11) Veröffentlichungsnummer : **0 007 972**
**B1**

# (12) EUROPÄISCHE PATENTSCHRIFT

(45) Veröffentlichungstag der Patentschrift :
**21.07.82**

(21) Anmeldenummer : **79101301.4**

(22) Anmeldetag : **30.04.79**

(51) Int. Cl.³ : **C 07 C109/04**

(54) **Verfahren zur Herstellung von Dichlorhydrazobenzolen durch katalytische Hydrierung von Monochlornitrobenzolen.**

(30) Priorität : **31.07.78 DE 2833605**

(43) Veröffentlichungstag der Anmeldung :
**20.02.80 (Patentblatt 80/04)**

(45) Bekanntmachung des Hinweises auf die Patenterteilung : **21.07.82 Patentblatt 82/29**

(84) Benannte Vertragsstaaten :
**BE CH DE FR GB NL**

(56) Entgegenhaltungen :
**EP - A - 0 000 519**
**FR - A - 2 360 563**
**US - A - 3 156 724**

(73) Patentinhaber : **DYNAMIT NOBEL AKTIENGESELL-SCHAFT**
**Patentabteilung Postfach 1209**
**D-5210 Troisdorf, Bez. Köln (DE)**

(72) Erfinder : **Herrmann, Hans-Joachim, Dr.**
**Ravensberger Weg 11**
**D-5210 Troisdorf (DE)**

Imprimerie Jouve, 18, rue St-Denis, 75001 Paris, France

Verfahren zur Herstellung von Dichlorhydrazobenzolen durch katalytische Hydrierung von Monochlornitrobenzolen

Gegenstand der vorliegenden Erfindung ist ein Verfahren zur Hydrierung von Monochlornitrobenzolen mit Wasserstoff zu Hydrazobenzolen bei Temperaturen von 40 bis 110 °C in Gegenwart eines Edelmetallkatalysators und vorzugsweise eines Cokatalysators und in Gegenwart einer wässrigen Lösung eines Alkalihydroxids und eines organischen Lösungsmittels, wobei das Lösungsmittel in turbulenter Bewegung gehalten wird, welches dadurch gekennzeichnet ist, daß man die Reaktion in einem organischen Lösungsmittel durchführt, in welchem das Dichlorhydrazobenzol in der Wärme gut und in der Kälte schlecht löslich ist, und daß man als wässrige Alkalihydroxidlösung im Falle von Natriumhydroxid eine 20 bis 40 Gew.-%ige Lösung und im Falle von Kaliumhydroxid eine 20 bis 38 Gew.-%ige Lösung einsetzt, und daß man den Edelmetallkatalysator in einer solchen Menge einsetzt, daß das Gewichtsverhältnis des eingesetzten Chlornitrobenzols zum Edelmetallkatalysator 1 : 0,000 05 bis 1 : kleiner als 0,000 2 ist.

Es ist bekannt, Hydrazobenzole von der Nitrostufe ausgehend durch Reduktion mit Zink und Alkali herzustellen (Alexejew, Zeitschrift für Chemie, *1868*, 497). Das in vieler Hinsicht nachteilige Arbeiten mit Zink kann durch Anwendung anderer Reduktionsmittel umgangen werden, jedoch lassen sich die angewandten Reduktionsmittel wie Dextrose (US-PS 2 794 047), Formaldehyd (US-PS 2 794 046), Hydrazinhydrat (DE-AS 2 609 530), Natriumsulfid (DE-OS 2 546 656) oder Natriumhydrogensulfid (BE-PS 832 268), Eisen und Säure (DE-OS 2 535 045), Natriumamalgam (DE-PS 1 668 896) technisch nur schwer handhaben und erfordern zudem meist gesondert hergestellte Azoxibenzole als Einsatzstoffe.

Die katalytische Hydrierung von Nitrobenzol im alkalischen Medium wird z.B. in der FR-PS 23 60 653 beschrieben. Die dort genannte Arbeitsweise ist zwar einfacher als die oben genannten Verfahren ; sie bedingt aber eine zuzätzliche Reinigungsoperation nach Abtrennung des Lösungsmittels, weil das dabei erhaltene Hydrazobenzol durch Anilin verunreinigt ist.

In der US-PS 3 156 724 wird ein Verfahren zur Herstellung von 2,2'-Dichlorhydrazobenzol beschrieben, bei welchem o-Chlornitrobenzol bei erhöhtem Druck und bei Temperaturen von 40 bis 100 °C mit Wasserstoff in Gegenwart eines Palladium- oder Platinkatalysators und einem Naphthochinon als Cokatalysator in wäßrig-alkalischem Medium und vorzugsweise in Gegenwart eines Lösungsmittels hydriert wird, das sowohl das Ausgangsprodukt als auch das Endprodukt löst. Als Lösungsmittel werden aromatische Kohlenwasserstoffe wie Toluol, Xylol oder Benzol vorgeschlagen. Nachteilig bei diesem bekannten Verfahren ist vor allem, daß zur Abtrennung des 2,2'-Dichlorhydrazobenzols von den unerwünschten Nebenprodukten umständliche Reinigungsoperationen erfoderlich sind. Dennoch wird auch danach nur ein mäßig reines 2,2'-Dichlorhydrazobenzol mit unbefriedigenden Ausbeuten als Destillationsrückstand erhalten. Nachteilig ist ferner, daß relativ große Mengen der Katalysatorkombination benötigt werden. (Das Gewichtsverhältnis Cokatalysator : o-Chlornitrobenzol soll im Bereich von 0,004 : 1 bis 0,008 : 1 liegen. Das bevorzugte Gewichtsverhältnis Edelmetallkatalysator zu o-Chlornitrobenzol wird mit 0,000 2 : 1 bis 0,001 : 1 angegeben). Gemäß den Angaben in dieser Patentschrift soll eine Reduzierung der Katalysatormenge eine Herabsetzung der Ausbeute zur Folge haben. Auch eine höhere Alkalität der Lösung, die mit maximal 20 % angegeben ist, soll ebenfalls die Ausbeute an Dichlorhydrazobenzol herabsetzen.

Unter den Hydrazobenzolen sind insbesondere die Dichlorhydrazobenzole von wirtschaftlicher Bedeutung. Im Vergleich mit unsubstituiertem Hydrazobenzol sind sie weit weniger gesundheitsgefährdend und daher handhabungssicherer. Unter den Dichlorhydrazoverbindungen gewinnt insbesondere das 2,2'-Dichlorhydrazobenzol steigendes Interesse, insbesondere als Zwischenprodukt bei der Herstellung von Farbpigmenten. Aber auch als Ausgangsprodukt zur Herstellung von Pharmazeutika ist das 2,2'-Dichlorhydrazobenzol von wirtschaftlicher Bedeutung.

An den Reinheitsgrad der Dichlorhydrazobenzole werden insbesondere dann sehr hohe Anforderungen gestellt, wenn sie als Zwischen- oder Vorprodukte für die Herstellung von Farbpigmenten eingesetzt werden sollen, weil sich Nebenprodukte, insbesondere durch Über- oder Unterreduktion entstandene Stoffe, bei der nachfolgenden Benzidin- oder Semidin-Umlagerung störend auswirken und dadurch die Qualität des Farbpigments nachteilig beeinflussen. Die bisher bekannten Reinigungsverfahren zur Abtrennung der in der Reaktion entstandenen Nebenprodukte befriedigen aber in der Praxis nicht. Man war nämlich bisher darauf angewiesen, das Reduktionsprodukt nach Abtrennung des Katalysators nehrmals mit wäßrigen Mineralsäuren zur Extraktion der stark basischen Nebenprodukte, wie Aniline, zu behandeln und in anschließenden weiteren Waschprozessen die überschüssige Mineralsäure und die entstandenen Salze zu entfernen. Trotz dieser mehrmaligen Waschprozesse gelingt es in den meisten Fällen nicht, Reinheitsgrade von mehr als 98 % zu erreichen. Zudem besteht dabei die Gefahr, daß eine unerwünschte Umlagerungsreaktion eintritt.

Der vorliegenden Erfindung liegt die Aufgabe zugrunde, ein technisch einfach zu handhabendes Verfahren zu schaffen, das die oben geschilderten Nachteile nicht aufweist und die gewünschten Hydrazobenzole in guter Qualität, insbesondere die Reinheit betreffend, bei gleichzeitig guten Ausbeuten liefert.

Die Aufgabe wird dadurch gelöst, daß man wie im Anspruch 1 angegeben arbeitet.

Bei dem erfindungsgemäßen Verfahren geht man so vor, daß man die als Einsatzstoffe verwendeten Nitrobenzole mit gasförmigem Wasserstoff und Edelmetallkatalysatoren in wäßrig-alkalischem Medium unter Zusatz des organischen Lösemittels und unter intensiver Durchmischung aller Phasen bis zum Erliegen der Wasserstoffaufnahme hydriert, den Edelmetallkatalysator auf mechanischem Wege, z.B. durch Filtrieren des warmen Gemisches entfernt, die das Hydrazobenzol enthaltende Phase in der Wärme abtrennt, das Dichlorhydrazobenzol unter Kühlung auskristallisieren läßt und es nach Filtrieren und Trocknen als Reinstoff gewinnt.

Als organische Lösungsmittel eignen sich gegen die Einwirkung von Wasserstoff bei den Reaktionsbedingungen inerte aliphatische und/oder cycloaliphatische Kohlenwasserstoffe, die nicht oder nicht in jedem Verhältnis mit Wasser mischbar sind. Im allgemeinen kommen bevorzugt geradkettige oder verzweigte gesättigte aliphatische und/oder cycloaliphatische Kohlenwasserstoffe in Frage, die bei der jeweiligen Reaktionstemperatur (bei Atmosphärendruck) als Flüssigkeit vorliegen. Geeignete Kohlenwasserstoffe der genannten Art sind vor allem $C_5$- bis $C_{12}$- Kohlenwasserstoffe.

Neben oder anstelle der Kohlenwasserstoffe können bei den Reaktionsbedingungen gegen Wasserstoff inerte primäre, sekundäre oder tertiäre, vorzugsweise primäre einwertige aliphatische oder cycloaliphatische Alkohole, insbesondere $C_4$ bis $C_8$-Alkohole eingesetzt werden, die nicht oder nicht in jedem Verhältnis mit Wasser mischbar sind.

Je nach den gewünschten Reaktionstemperaturen werden bevorzugt solche Lösungsmittel eingesetzt, deren Dampfdruck bei der Reaktionstemperatur 1 bar nicht überschreitet.

Eine weitere Forderung für zu verwendende Lösungsmittel ist, daß sie auf einfache Weise zurückgewinnbar sein sollten, damit sie in den Reduktionsprozeß zurückgeführt werden können. Als besonders geeignete Kohlenwasserstoffe seien die $C_6$ bis $C_{10}$-Kohlenwasserstoffe hervorgehoben mit Siedepunkten ab 68,7 °C (Hexan) bis 174,1 °C (Decan). Geeignete Lösungsmittel sind aber auch Erdölfraktionen wie Petroläther, Leichtbenzin, Schwerbenzin, Ligroin und dgl.

Von den cycloaliphatischen Verbindungen seien als bevorzugte Vertreter genannt die $C_5$- bis $C_8$-Kohlenwasserstoffe.

Bei dem erfindungsgemäßen Verfahren werden bevorzugt n-Heptan und/oder Cyclohexan eingesetzt.

Unter den genannten Alkoholen wird n-Butanol bevorzugt.

Das Gewichtsverhältnis des Lösemittels zu dem eingesetzten Nitrobenzol beträgt im allgemeinen 0,3 bis 4 : 1, bevorzugt 1,5 : 1, kann aber auch je nach dem Löslichkeitsverhalten gegenüber dem Nitrobenzol, (denn dieses soll während der Hydrierung in Form einer Lösung oder Schmelze vorliegen), und gegenüber dem Dichlorhydrazobenzol, das beim Abkühlen der organischen Phase auskristallisieren soll, auch oberhalb oder unterhalb dieses Bereiches liegen.

Als Alkalihydroxide werden erfindungsgemäß Natriumhydroxid oder Kaliumhydroxid eingesetzt. Die wäßrig-alkalische Lösung dieser Hydroxide sollte zweckmäßig einen pH-Wert von wenigstens 12 aufweisen. Das bevorzugte Hydroxid ist Natriumhydroxid.

Die Konzentrationen der erfindungsgemäß eingesetzten Alkalihydroxide liegen zwischen 20 und 40 Gew.-%. Im Falle von Natriumhydroxid wird eine 20 bis 40 Gew.-%-ige Lösung und im Falle von Kaliumhydroxid eine 20 bis 38 Gew.-%-ige Lösung eingesetzt. Es hat sich nämlich überraschenderweise entgegen der Lehre der US-PS 3 156 724, Spalte 2, Zeilen 23 bis 25 herausgestellt, daß die besten Ergebnisse bei Anwendung von größer als 20 Gew.-%-igen alkalischen Lösungen erhalten werden.

Bevorzugt wird mit solchen Anteilen an wäßriger Lauge gearbeitet, daß die Laugenkonzentration zu Beginn der Reduktion bei einem hohen Wert von 38 bis 40 Gew.-%, am Ende der Reduktion nach Verdünnung mit dem in der Reaktion entstandenen Wasser dagegen zwischen 14 und 27 Gew.-%, vorzugsweise zwischen 18 und 27 Gew.-% liegt. Diese Arbeitsweise hat eine verringerte Bildung an Chloranilin zur Folge.

Im allgemeinen liegen die Gewichtsverhältnisse der eingesetzten wäßrig-alkalischen Lösungen zu den eingesetzten Nitrobenzolen zwischen 0,2 : 1 und 2,6 : 1, bevorzugt zwischen 0,5 bis 0,8 : 1.

Zur besseren Emulgierung von wäßriger und organischer Phase können geringe Mengen von Emulgatoren zugesetzt werden, z.B. Sulfonsäureester langkettiger aliphatischer Alkohole.

Als Katalysatoren werden Edelmetallkatalysatoren, bevorzugt auf inertem Träger, wie Palladium, Platin und Rhodium, einzeln oder im Gemisch auf Aktivkohle, Aluminiumoxid, Titandioxid oder Silicagel eingesetzt. Handelsübliche Edelmetallkatalysatoren weisen im allgemeinen Metallgehalt von 0,5 bis 20 Gew.-% auf. Bevorzugt werden Katalysatoren mit 1 bis 5 % Edelmetallgehalt zur Anwendung gebracht. Bevorzugt eingesetzte Katalysatoren sind im Handel erhältliche Typen mit 5 Gew.-% Platin in reduzierter Form auf Altivkohle und einem Schüttgewicht zwischen 250 bis 300 g/l, vorzugsweise von ca. 270 g/l, einer Korngrößenverteilung zwischen 5 und 60 µ (80 Gew.-%), vorzugsweise zwischen 5 und 40 µ, einer spezifischen Gesamtoberfläche nach BET von ca. 700 bis 1000 m²/g, vorzugsweise ca. 800 m²/g, einer spezifischen Metalloberfläche von 15 bis 25 m²/g, vorzugsweise um 20 m²/g und einem Aschegehalt unter 0,1 Gew.-%.

Bevorzugt werden solche Katalysatoren, die sich auf mechanischem Wege, z.B. durch Filtration oder Zentrifugieren, von der Reaktionsmischung leicht und vollständig abtrennen lassen. Vor allem derartige Katalysatoren lassen sich nach dem Gebrauch gut auswaschen, z.B. mit Lösemitteln wie Aceton oder Methanol, und wieder in einen gebrauchsfähigen Zustand überführen.

Die im Anspruch angegebene Menge Edelmetallkatalysator bezieht sich auf das Gewicht des reinen Metalls. Die Korngrößen der bevorzugt eingesetzten Edelmetallkatalysatoren liegen im allgemeinen im Bereich zwischen 0 und 60 μ.

Als Cokatalysatoren eignen sich Chinone, wie beispielsweise p-Benzochinon, Hydrochinon, 1,4-Naphthochinon, 1,4-Naphthalindiol, 5,6,7,8-Tetra-hydro-naphthochinon-1,4,2,3-Dichlor-naphtochinon-1,4 und dgl. Bevorzugt werden die Vertreter der Naphthalinreihe eingesetzt, insbesondere 1,4-Naphthochinon.

Das Gewichtsverhältnis der Cokatalysatoren zum eingesetzten Nitrobenzol liegt zwischen 0,000 5 : 1 und kleiner 0,002 : 1, im bevorzugten Fall zwischen 0,000 5 : 1 und 0,001 5 : 1. Ohne Cokatalysator werden relativ hohe Anteile an Chloranilinen erhalten. Zusätzlich besteht die Gefahr, daß Enthalogenierung auftritt.

Der Wasserstoffdruck kann während der Reduktion in weiten Grenzen gehalten werden, die von 0,5 bar bis 30 bar und mehr reichen; bevorzugt wird bei 6 bis 12 bar gearbeitet, wobei der höhere Druck insbesondere gegen Ende der Reduktion vorteilhaft ist. Bevorzugt arbeitet man daher bei ansteigendem, vorzugsweise stetig ansteigendem Druck.

Die Reaktion läuft im Temperaturbereich von 40 bis 110 °C ab. Bevorzugt liegt sie bei 55 bis 75 °C, wo die Reaktionsgeschwindigkeit schon genügend hohe Werte annimmt und andererseits die Gefahr der Nebenproduktbildung infolge Enthalogenierung oder Überreduktion noch gering ist. Die freiwerdende erhebliche Reaktionswärme wird durch Kühlung abgeführt, damit die Temperaturkonstanz des Reaktionsgefäßes erhalten bleibt.

Es ist vorteilhaft, im Reaktionsraum, z.B. einem Autoklaven, eine größtmögliche Stoffaustauschfläche zu schaffen, indem man alle Phasen in intensiven gegenseitigen Kontakt bringt. Dies kann durch Verwendung von wirksamen Stoffaustauschapparaten wie Begasungsrührern, Umlaufreaktoren mit stromstörenden Einbauten, Düsen oder dgl. bewerkstelligt werden.

Eine besonders bevorzugte Vorrichtung, die zur Durchführung des erfindungsgemäßen Verfahrens eingesetzt werden kann, ist in der deutschen Patentanmeldung P 27 08 914.1 (OZ : 77019) beschrieben. Wesentliche Bestandteile dieser Hydriervorrichtung sind ein temperierbarer Reaktor, eine Pumpe, eine Zerstäuberdüse, z.B. nach Art der Venturi-Düse oder Wasserstrahlpumpe, sowie Temperatur- und Druckregler. Über einen Umlauf wird das Reaktionsgut über eine Düse zusammen mit Wasserstoff in den Reaktor zurückgepumpt.

Der Reaktor ist vertikal angeordnet und so dimensioniert, daß mindestens 10 bis 20 % seines Volumens als Gasraum zur Verfügung stehen. Der mit einer Pumpe versehene Umlauf führt zu der Düse, welche im oberen Teil des Reaktors im Bereich des Gasraums angebracht ist und in den Wasserstoffgasraum des Reaktors hineinreicht. Die Zuleitung von Wasserstoff in die Düse kann über einen seitlichen Ansaugstutzen erfolgen, welchem laufend Wasserstoff zugeführt wird. Nach beendeter Hydrierung wird das Reaktionsgemisch, bevorzugt unter Stickstoffatmosphäre, dem Reaktor entnommen und heiß filtriert.

Die Reaktionsdauer beträgt im allgemeinen 2 bis 6 Stunden, kann jedoch auch darüber oder darunter liegen, in Abhängigkeit von den Drücken, Temperaturen und der Katalysatormenge. In dieser Zeit setzen sich die eingesetzten Nitrobenzole weitgehend über die meist faßbare Zwischenstufe der Azoxibenzole zu den Hydrazobenzolen als Zielprodukt um. Der Anteil der durch Überreduktion entstehenden Aniline ist relativ gering. Nach beendeter Reaktion, gekennzeichnet durch zum Stillstand kommende Wasserstoffaufnahme und abklingende Wärmeetwicklung, wird die Reaktionsmischung heiß filtriert oder zentrifugiert zwecks Abtrennung der Feststoffe, insbesondere der Katalysatoren. Im nächsten Schritt wird die heiße wäßrig-alkalische Phase, die sich im allgemeinen als schwere Phase unten absetzt, von der heißen organischen Phase getrennt, z.B. durch Ablassen. Letzere wird abgekühlt, z.B. in wassergekühlten Gefäßen, wobei das Dichlorhydrazobenzol auskristallisiert. Nach Beendigung der Kristallisation wird das Kristallisat auf mechanischem Wege, z.B. durch Filtration, von der Mutterlauge getrennt. Nach dem Trocknen, vorzugsweise unter Anlegen eines Vakuums, erhält man die Hydrazobenzole als reine Stoffe.

Bei einer bevorzugten Ausführungsform des erfindungsgemäßen Verfahrens wird die Hydrierung und die Abtrennung der Feststoffe sowie der wäßrig-alkalischen Lösung im gleichen oder annähernd gleichen Temperaturbereich durchgeführt.

Bei einer weiteren bevorzugten Ausführungsform setzt man als Nitrobenzol o-Chlornitrobenzol zur Hydrierung ein.

Das Lösemittel, das den Hauptanteil der organischen Mutterlauge bilder, läßt sich durch Destillation wiedergewinnen und in den Prozeß zurückführen. Sollten sich während der Reduktion Aniline gebildet haben, finden sie sich im Rückstand nach dem Abdestillieren des Lösemittels und können durch Destillieren bei erhöhter Temperatur und/oder Vakuum gewonnen werden. Der dann noch verbleibende Rückstand kristallisiert beim Erkalten unter Zusatz von Lösemittel und liefert eine zweite Fraktion des Hydrazobenzols. Als Lösemittel wird zweckmäßig das gleiche Lösemittel verwendet, das auch zur Reduktion eingesetzt wurde.

Die zuvor abgetrennte wäßrig-alkalische Mutterlauge wird durch Andestillieren von geringen Mengen an gelöstem oder emulgiertem Lösemittel befreit, unter Zusatz von Aktivkohle erhitzt, vorzugsweise auf den Siedepunkt, und vorzugsweise unter Einblasung von Luft, wobei das noch vor handene organische Material größtenteils als Schlamm ausfällt und die ursprüngliche Laugekonzentration durch Abdestillieren des bei der Reduktion gebildeten Wassers wiederhergestellt wird. Nach dem Filtrieren erhält man eine Lauge, die ca. 10 mal wieder zur Reduktion verwendbar ist.

Das beschriebene Verfahren weist in mehrerer Hinsicht Vorteile gegenüber bekannten Verfahren auf.

Man gelangt direkt von der Nitrostufe zu den gewünschten Hydrazobenzolen, ohne gesondert hergestellte Zwischenstufen einsetzen zu müssen. Die Zielprodukte fallen in guten Ausbeuten und mit hohen Reinheiten an. Dies ist bei Verwendung zur Herstellung der in der Farbstoffsynthese gebrauchten Benzidine von großem Nutzen, da ihre Qualität in hohem Maße von der Reinheit der eingesetzten Hydrazobenzole abhängt. Zur Reduktion der Nitrobenzole sind nur relativ geringe Mengen an Edelmetallkatalysatoren ggf. in gut handhabbaren wasserfeuchtem Anlieferungszustand, notwendig, die zudem wiedergewonnen und direkt oder nach Umarbeitung durch den Hersteller erneut einsetzbar sind. Trotz dieser kleinen Mengen läuft die Reaktion, unabhängig von der Größe des Reaktionsansatzes, in befriedigend kurzer Zeit ab. Alle Hilfsstoffe, insbesondere das organische Lösungsmittel und das wäßrige Alkali, werden nach Durchlaufen von Reinigungsstufen in den Prozeß zurückgeführt, so daß nur durch Destillation gereinigtes Reaktionswasser als die Umwelt nicht belastendes Nebenprodukt anfällt. Insgesamt finden sich im beschriebenen Verfahren einfach zu beherrschende Teilschritte, wodurch das Verfahren handhabungssicher und damit betriebssicher ist.

Beispiel 1

630 g m-Chlornitrobenzol, 450 g Wasser und 135 g NaOH (entspricht einer 23,1 Gew.-%-igen wäßrigen Lösung), 0,7 g Naphthalindiol-1,4, 500 g Cyclohexan und 0,7 g Palladium auf Aktivkohle, 5 % Pd (das Mengenverhältnis an Pd-Metall zum eingesetzten m-Chlornitrobenzol beträgt 0,000 055 : 1) werden bei 65 bis 75 °C unter 7 bar Wasserstoffdruck und intensivem Rühren im Autoklaven hydriert. Nach 3 bis 3 1/2 Studen ist die Reduktion beendet, dadurch erkennbar, daß keine Wasserstoffaufnahme mehr erfolgt. Man filtriert den Katalysator bei 75 bis 80 °C ab, läßt absitzen, bis sich die beiden verbleibenden flüssigen Phasen vollständig getrennt haben, entfernt die schwere wäßrige Phase durch Ablassen bei 75 bis 80 °C und führt die obere, das Reduktionsprodukt enthaltende Phase der Kristallisation zu. Nach Abkühlen auf 20 °C wird das ausgefallene 3,3'-Dichlorhydrazobenzol abfiltriert, mit ca. 100 g kaltem Cyclohexan bis zum Verschwinden der Gelbfärbung gewaschen und im Vakuum bei 60 °C getrocknet. Man erhält 477 g 3,3-Dichlorhydrazobenzol vom Schmelzpunkt 94 °C, entsprechend einer Ausbeute von 94 % der Theorie, welches eine Reinheit von etwa 99 % aufweist (flüssigkeitschromatographisch bestimmt). Das Nebenprodukt m-Chloranilin entsteht nur zu einem Anteil von 1,3 bis 1,4 Gew.-%.

Beispiel 2

210 g p-Chlornitrobenzol, 600 g Wasser und 180 g Natriumhydroxid (entspricht einer 23,1 Gew.-%-igen wäßrigen Lösung), 0,3 g 2,3-Dichlornaphthochinon-1,4, 350 g n-Hexan und 0,40 g Platin auf Aktivkohle, 5 % Pt (das Mengenverhältnis an Pt-Metall zum eingesetzten p-Chlornitrobenzol beträgt 0,000 095 : 1) werden mittels Begasungsrührer bei 6 bar Wasserstoffdruck und 60 bis 65 °C hydriert. Nach 4 bis 5 Stunden ist die Reduktion beendet. Man arbeitet wie unter Beispiel 1 beschrieben auf und erhält 151 g 4,4'-Dichlorhydrazobenzol vom Schmelzpunkt 120-121 °C und einer Reinheit von 98,5 %, was einer Ausbeute von 88,5 % der Theorie entspricht. Das mögliche Nebenprodukt p-Chloranilin wurde nicht nachgewiesen.

Beispiel 3

630 g o-Chlornitrobenzol, 615 g Wasser und 185 g Natriumhydroxid (entspricht einer 23,1 Gew.-%-igen wäßrigen Lösung), 0,9 g Naphthalindiol-1,4, 535 g n-Butanol und 1,0 g Palladium auf Aluminiumoxid, 5 % Pd (das Mengenverhältnis an Pd-Metall zum eingesetzten o-Chlornitrobenzol beträgt 0,000 08 : 1) werden bei 6 bar Wasserstoffdruck und 55 bis 65 °C während 4 Stunden hydriert und wie in Beispiel 1 beschrieben aufgearbeitet. Man erhält 396 reines 2,2'-Dichlorhydrazobenzol (Reinheit größer als 98 %) mit einer Ausbeute von 78 % der Theorie, daneben fallen 37 g o-Chloranilin an, die nach dem Abziehen des n-Butanols im Vakuum destillierbar sind und so rein gewonnen werden können. Wird der Destillationsrückstand mit 65 g n-Butanol von 60 °C versetzt, so kristallisiert beim Abkühlen eine zweite Fraktion von 61 g 2,2-Dichlorhydrazobenzol aus, deren Reinheit 97 bis 98 % beträgt. Die Gesamtausbeute an Hydrazobenzol liegt somit bei 89,8 %, daneben entstehen 7,3 Gew.-% o-Chloranilin.

Beispiel 4

63 kg o-Chlornitrobenzol, 31,1 kg Wasser und 13,85 kg 86,7 Gew.-%-iges Kaliumhydroxid (entspricht einer 26,8 Gew.-%-igen wäßrigen Lösung), 65 kg n-Heptan, 92 g 1,4-Naphthochinon und 350 g Pt-Kohle, 1 % Pt (das Mengenverhältnis an Pt-Metall zum eingesetzten o-Chlornitrobenzol beträgt 0,000 055 : 1) werden bei 6 bar Wasserstoffdruck und 55 bis 65 °C in einem Umlaufreaktor während 4 Stunden hydriert und wie im Beispiel 3 beschrieben aufgearbeitet. Man erhält zwei Fraktionen an 2,2-Dichlorhydrazobenzol von 39 und 7,5 kg, entsprechend einer Gesamtausbeute an Hydrazobenzol von 91,5 % der Theorie sowie 3,2 kg o-Chloranilin, entsprechend einem Anteil von 6,3 %. Die Reinheiten der Hydrazobenzol-Fraktionen lagen über 99 % (Fraktion 1) bzw. größer als 98 % (Fraktion 2), flüssigkeits-chromatographisch bestimmt.

**Ansprüche**

1. Verfahren zur Hydrierung von Mono-chlornitrobenzolen mit Wasserstoff zu Di-chlorhydrazobenzolen bei Temperaturen von 40 bis 110 °C in Gegenwart eines Edelmetallkataly-sators und vorzugsweise eines Cokatalysators und in Gegenwart einer wäßrigen Lösung eines Alkali-hydroxids und eines organischen Lösungsmittel-s, wobei das Reaktionsgemisch in turbulenter Bewegung gehalten wird, dadurch gekennzeichnet, daß man die Reaktion in einem organischen Lösungsmittel durchführt, in welchem das Dichlorhydrazobenzol in der Wärme gut und in der Kälte schlecht löslich ist und daß man als Alkali-hydroxidlösung im Falle von Natriumhydroxid eine 20 bis 40 Gew.-%-ige Lösung und im Falle von Kaliumhydroxid eine 20 bis 38 Gew.-%-ige Lösung einsetzt, und daß man den Edelmetallkatalysator in einer solchen Menge einsetzt, daß das Ge-wichtsverhältnis des eingesetzten Chlornitro-benzols zum Edelmetallkatalysator 1 : 0,000 05 bis 1 : kleiner als 0,000 2 ist.

2. Verfahren gemäß Anspruch 1, dadurch gekennzeichnet, daß das Gewichtsverhältnis des eingesetzten Chlornitrobenzols zum Edelmetall-katalysator zwischen 1 : 0,000 05 bis 1 : < 0,000 1 beträgt.

3. Verfahren nach Anspruch 1 oder 2, dadurch gekennzeichnet, daß das Gewichtsverhältnis von Monochlornitrobenzol zum Cokatalysator 1 : 0,000 5 bis 1 : < 0,002, vorzugsweise 1 : 0,000 5 bis 1 : 0,001 5 ist.

4. Verfahren nach einem der Ansprüche 1 bis 3, dadurch gekennzeichnet, daß der Cokatalysator ein Chinon der Naphthalinreihe ist.

5. Verfahren nach Anspruch 4, dadurch gekennzeichnet, daß das Chinon 1,4-Naphthochinon ist.

6. Verfahren nach einem der Ansprüche 1 bis 5, dadurch gekennzeichnet, daß das organische Lösungsmittel nicht oder nicht in jedem Verhältnis mit Wasser mischbar ist.

7. Verfahren nach Anspruch 6, dadurch gekennzeichnet, daß als Lösungsmittel gegen die Einwirkung von Wasserstoff bei den Reaktionsbedingungen inerte aliphatische und/oder cyclo-aliphatische Kohlenwasserstoffe eingesetzt wer-den.

8. Verfahren nach Anspruch 7, dadurch gekennzeichnet, daß das Lösungsmittel n-Heptan und/oder Cyclohexan ist.

9. Verfahren nach Anspruch 6, dadurch gekennzeichnet, daß als Lösungsmittel bei den Reaktionsbedingungen gegen Wasserstoff inerte primäre, sekundäre oder tertiäre, vorzugsweise primäre einwertige aliphatische oder cycloalipha-tische Alkohole, insbesondere $C_4$ bis $C_8$-Alkoho-le, eingesetzt werden.

10. Verfahren nach Anspruch 9, dadurch gekennzeichnet, daß der Alkohol n-Butanol ist.

11. Verfahren nach einem der Ansprüche 1 bis 10, dadurch gekennzeichnet, daß die Feststoffe nach beendeter Hydrierung auf mechanischem Wege in der Wärme abgetrennt werden und daß — nach dem Abtrennen der wässrig-alkalischen Phase aus dem Reaktionsgemisch in der Wärme — das Hydrazobenzol durch Kühlungskristallisation aus der organischen Phase gewonnen wird.

12. Verfahren nach Anspruch 11, dadurch gekennzeichnet, daß die Hydrierung und die Abtrennung der Feststoffe sowie der wässrig-alkalischen Lösung im gleichen oder annähernd gleichen Temperaturbereich durchgeführt werden.

13. Verfahren nach einem der Ansprüche 1 bis 12, dadurch gekennzeichnet, daß als Nitrobenzol o-Chlornitrobenzol eingesetzt wird.

**Claims**

1. Process for the hydrogenation of mono-chloronitrobenzenes with hydrogen to form di-chlorohydrazobenzenes at temperatures of 40 to 110 °C in the presence of a noble metal catalyst and preferably a co-catalyst and in the presence of an aqueous solution of an alkalihydroxide and an organic solvent, wherein the reaction mixture is kept in turbulent motion, characterised in that the reaction is carried out in an organic solvent in which the dichlorohydrazobenzene has good solubility on warming and poor solubility in the cold and that, in the case of sodium hydroxide there is used as alkalihydroxide solution a 20 to 40 % by weight solution and in the case of potassium hydroxide a 20 to 38 % by weight solution, and that the noble metal catalyst is employed in such amount that the weight ratio of the chloronitrobenzene employed to the noble metal catalyst is 1 : 0.000 05 to 1 : less than 0.000 2.

2. Process according to claim 1, characterised in that the weight ratio of the chloronitrobenzene employed to the noble metal catalyst amounts to between 1 : 0.000 05 to 1 : < 0,000 1.

3. Process according to claim 1 or 2, characterised in that the weight ratio of monochloroni-trobenzene to co-catalyst is 1 : 0.000 5 to 1 : < 0.002, preferably 1 : 0.000 5 to 1 : 0.001 5.

4. Process according to one of claims 1 to 3, characterised in that the co-catalyst is a quinone of the naphthalene series.

5. Process according to claim 4, characterised in that the quinone is 1,4-naphthoquinone.

6. Process according to one of claims 1 to 5, characterised in that the organic solvent is not miscible with water in any ratio.

7. Process according to claim 6, characterised in that inert aliphatic and/or cycloaliphatic hydrocarbons are employed as solvents inert against the action of hydrogen under the reaction conditions.

8. Process according to claim 7, characterised in that the solvent is n-heptane and/or cyclohexane.

9. Process according to claim 6, characterised in that primary, secondary or tertiary, preferably primary, monofunctional aliphatic or cycloaliphatic alcohols, especially $C_4$ to $C_8$-alcohols are employed as solvents inert to hydrogen under the reaction conditions.

10. Process according to claim 9, characterised in that the alcohol is n-butanol.

11. Process according to one of claims 1 to 10, characterised in that the solid materials after the termination of hydrogenation are separated off in mechanical manner at elevated temperature and that — after the separation off of the aqueous-alkali phase from the reaction mixture at elevated temperature — the hydrazobenzene is recovered from the organic phase by crystallisation under cooling.

12. Process according to claim 11, characterised in that the hydrogenation and the separation off of the solid materials as well as of the aqueous-alkali solution are carried out in the same or approximately the same temperature region.

13. Process according to one claims 1 to 12, characterised in that o-chloronitrobenzene is employed as the nitrobenzene.

**Revendications**

1. Procédé pour l'hydrogénation de monochloronitrobenzènes en dichlorohydrazobenzènes avec de l'hydrogène à des températures comprises entre 40 et 110 °C en présence d'un catalyseur au métal noble et de préférence d'un cocatalyseur et en présence d'une solution aqueuse d'un hydroxyde alcalin et d'un solvant organique, dans lequel on maintient le mélange réactionnel en mouvement turbulent, caractérisé en ce qu'on conduit la réaction dans un solvant organique dans lequel le dichlorohydrazobenzène est bien soluble à chaud et peu soluble à froid, et on utilise en tant que solution d'hydroxyde alcalin une solution de 20 à 40 % en poids dans le cas d'hydroxyde de sodium et une solution de 20 à 38 % en poids dans le cas d'hydroxyde de potassium, et on utilise le catalyseur au métal noble en une quantité telle que le rapport pondéral du chloronitrobenzène utilisé au catalyseur de métal noble soit compris entre 1 : 0,000 05 et 1 : moins de 0,000 2.

2. Procédé selon la revendication 1, caractérisé en ce que le rapport pondéral du chloronitrobenzène utilisé au catalyseur au métal noble est compris entre 1 : 0,000 05 et 1 : moins de 0,000 1.

3. Procédé selon l'une des revendications 1 ou 2, caractérisé en ce que le rapport pondéral du monochloronitrobenzène au cocatalyseur est compris entre 1 : 0,000 5 et 1 : moins de 0,002, de préférence entre 1 : 0,000 5 et 1 : 0,001 5.

4. Procédé selon l'une quelconque des revendications 1 à 3, caractérisé en ce que le cocatalyseur est une quinone de la série du naphtalène.

5. Procédé selon la revendication 4, caractérisé en ce que la quinone est la 1,4-naphtoquinone.

6. Procédé selon l'une quelconque des revendications 1 à 5, caractérisé en ce que le solvant organique n'est pas miscible à l'eau ou n'est pas miscible à l'eau en toutes proportions.

7. Procédé selon la revendication 6, caractérisé en ce qu'on utilise à titre de solvant des hydrocarbures aliphatiques et/ou cycloaliphatiques inertes vis-à-vis de l'action de l'hydrogène dans les conditions de réaction.

8. Procédé selon la revendication 7, caractérisé en ce que le solvant est le n-heptane et/ou le cyclohexane.

9. Procédé selon la revendication 6, caractérisé en ce qu'on utilise à titre de solvant des monoalcools aliphatiques ou cycloaliphatiques primaires, seondaires ou tertiaires, inertes vis-à-vis de l'hydrogène dans les conditions de réaction, de préférence des monoalcools primaires, en particulier des alcools en $C_4$ à $C_8$.

10. Procédé selon la revendication 9, caractérisé en ce que l'alcool est le n-butanol.

11. Procédé selon l'une quelconque des revendications 1 à 10, caractérisé en ce que les substances solides sont séparées à chaud par un moyen mécanique après la fin de l'hydrogénation et — après la séparation de la phase alcaline aqueuse du mélange réactionnel à chaud — l'hydrazobenzène est récupéré par cristallisation à froid à partir de la phase organique.

12. Procédé selon la revendication 11, caractérisé en ce que l'hydrogénation et la séparation des substances solides, ainsi que de la solution alcaline aqueuse, sont effectuées dans une gamme de températures identiques ou à peu près identiques.

13. Procédé selon l'une quelconque des revendications 1 à 12, caractérisé en ce que comme nitrobenzène on utilise l'o-chloronitrobenzène.